# EUROPEAN PATENT APPLICATION

(11) **EP 1 215 192 A2**
(43) Date of publication of application: **19.06.2002**
(21) Application number: 01129634.0
(22) Date of filing: 12.12.2001
(51) Int. Cl.: C07C 51/353, C07C 67/347, C07D 303/16, C07C 57/50, C07C 69/618, C07C 69/616

(54) **Preparation process of dicarboxylic acid compounds**

(30) Priority: 14.12.2000 JP 2000380233
(71) Applicant: Adchemco Corporation, Tokyo 102-0073 (JP)
(72) Inventor: Mori, Hiroaki, c/o Adchemco Corporation, Tokyo 102-0073 (JP)
(74) Representative: Wächtershäuser, Günter, Prof. Dr.

(57) **Abstract**

Carboxylic acid compounds, each of which has an indene skeleton and represented by the following formula (2) or a fluorene skeleton and represented by the following formula (3), are each prepared by reacting an unsaturated carboxylic acid compound represented by the following formula (1) with indene or fluorene in the presence of a strong base catalyst: 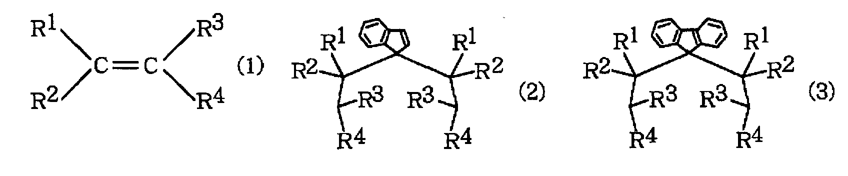 wherein R¹, R², R³ and R⁴ each independently represents an atom or group selected from a hydrogen atom, an alkyl group, an aryl group or a carboxyl group, with the proviso that at least one of R³ and R⁴ contains a carboxyl group or a salt thereof or a carboxylic ester group.

## Description

This invention relates to a process for the preparation of carboxylic acid compounds having the indene or fluorene skeleton.

Carboxylic acid compounds having the indene or fluorene skeleton are useful as monomers for high functional polymers such as polyesters, polyamides and epoxy resins. The term "carboxylic acid compounds" as used herein should be construed to encompass therein compounds having one or more groups such as free carboxyl groups or salts or esters thereof.

It has been a conventional practice to synthesize a carboxylic acid compound, which has the indene or fluorene skeleton, by the process taught by Bruson in U.S. Patent No. 2,280,058, that is, by causing acrylonitrile to act on an active-hydrogen-containing compound such as indene or fluorene in the presence of a base catalyst (Triton B) to obtain the corresponding dicyanoethyl derivative, hydrolyzing the dicyanoethyl derivative, and then going through an esterification step. This process is, however, not fully considered to be an advantageous process from the economical standpoint, because two steps are required to obtain the target carboxylic acid, three steps are needed to obtain its ester, and the overall yield through all the steps is low.

An object of the present invention is, therefore, to provide an industrially advantageous process for the preparation of a carboxylic acid compound having the indene or fluorene skeleton, the industrially advantageous preparation of which has not been satisfactorily achieved by any conventional processes.

The above-described object can be achieved by the present invention as will be described hereinafter.

In one aspect of the present invention, there is thus provided a process for the preparation of a carboxylic acid compound having an indene skeleton and represented by the following formula (2), characterized by reacting an unsaturated carboxylic acid compound represented by the following formula (1) with indene in the presence of a strong base catalyst: 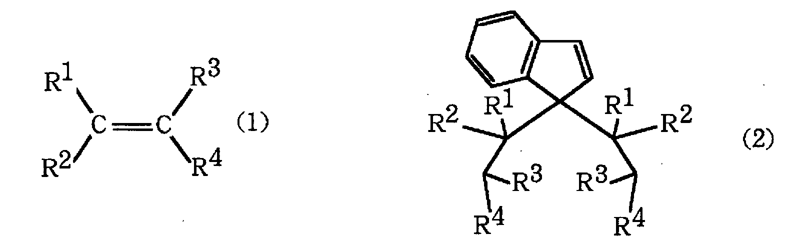 wherein R¹, R², R³ and R⁴ each independently represents an atom or group selected from a hydrogen atom, an alkyl group, an aryl group or a carboxyl group, with the proviso that at least one of R³ and R⁴ contains a carboxyl group or a salt thereof or a carboxylic ester group.

In another aspect of the present invention, there is also provided a process for the preparation of a carboxylic acid compound having a fluorene skeleton and represented by the following formula (3), characterized by reacting an unsaturated carboxylic acid compound represented by the following formula (1) with fluorene in the presence of a strong base catalyst: 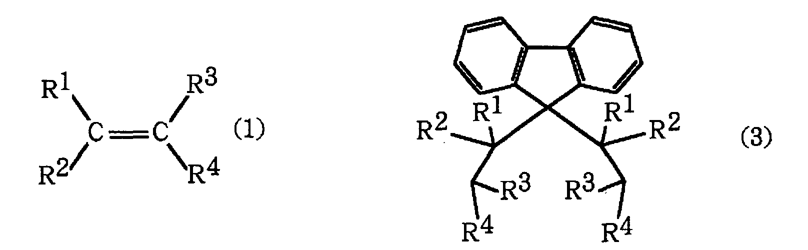 wherein R¹, R², R³ and R⁴ each independently represents an atom or group selected from a hydrogen atom, an alkyl group, an aryl group or a carboxyl group, with the proviso that at least one of R³ and R⁴ contains a carboxyl group or a salt thereof or a carboxylic ester group.

In the definition of R³ and R⁴, at least one of the alkyl group and aryl group may preferably contain an oxygen-containing group selected from a carboxyl group, a hydroxyl group or an ether group.

According to the present invention, the carboxylic acid compound having the indene or fluorene skeleton and represented by the formula (2) or (3) can be synthesized directly. Therefore, the process according to the present invention can solve the above-described conventional problems, and can prepare the carboxylic acid compound having the indene or fluorene skeleton on an industrial scale with excellent economy.

The present invention will next be described in further detail based on certain preferred embodiments.

As indene or fluorene for use in the present invention, it is possible to use indene or fluorene obtained from a raw material of petroleum origin or coal origin through separation and purification steps. Purified indene or fluorene is usable irrespective of its purity insofar as no other active-hydrogen-containing compound is contained as an impurity, although one having a purity of 90 wt.% or higher is preferred.

No particular limitation is imposed on the unsaturated carboxylic acid compound for use in the present invention insofar as it is represented by the below-described formula (1). Particularly preferred examples of the unsaturated carboxylic acid compound can include (meth)acrylic acid, itaconic acid, cinnamic acid and maleic acid, fumaric acid and their salts and C₁-C₄ mono- or dialkyl esters. The term "(meth) acrylic acid" as used herein should be interpreted to embrace therein both acrylic acid and methacrylic acid. 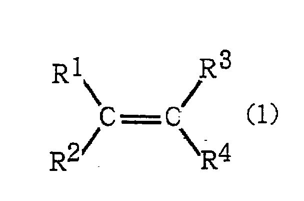 wherein R¹, R², R³ and R⁴ have the same meanings as defined above.

Illustrative of the (meth)acrylic esters are methyl (meth)acrylate, ethyl (meth)acrylate, propyl (meth)acrylate and glycidyl (methacrylate), illustrative of the itaconic esters are itaconic mono- or diesters such as dimethyl itaconate and mono- or diethyl itaconate, illustrative of the cinnamic esters are methyl cinnamate and ethyl cinnamate, and illustrative of maleic or fumaric esters are maleic or fumaric mono- or diesters such as mono- or dimethyl maleate or fumarate and mono- or diethyl maleate or fumarate.

When indene is used as a starting raw material, the unsaturated carboxylic acid compound represented by the formula (1) can be used in a proportion of from 0.05 to 4 moles, preferably from 0.1 to 2 moles per mole of the indene. Use of the unsaturated carboxylic acid compound represented by the formula (1) in a proportion greater than the above-described range is not preferred, because addition to the unsaturated carbon atoms on the 5-membered ring of indene is also induced. When fluorene is used as a starting raw material, the unsaturated carboxylic acid compound can be used in a proportion of from 2 to 10 moles, preferably from 3 to 6 moles per mole of the fluorene.

Examples of the strong basic catalyst for use in the present invention can include quaternary ammonium hydroxides such as benzyltrimethylammonium hydroxide (Triton B) and benzyltriethylammonium hydroxide; alkali metal alkoxides such as sodium methoxide, sodium ethoxide and potassium tert-butoxide; and mixtures thereof. The strong base catalyst can be used in a proportion of from 0.5 to 10 mole %, preferably from 1 to 5 mole % based on the indene or fluorene. A combined use of a quaternary ammonium hydroxide and an alkali metal alkoxide is preferred. As their mixing proportions, it is preferred to use the latter in a proportion of from about 0.1 to 5 moles per mole of the former.

Upon practice of the process according to the present invention, a solvent is used in general. Suitable examples of such a solvent can include ether solvents such as tetrahydrofuran, dioxane, ethylene glycol dimethyl ether.

In the present invention, the reaction temperature may generally range from 0 to 100°C, with a range of from 10 to 50°C being suited. The reaction time, on the other hand, may generally range from 3 to 50 hours, with a range of from 20 to 40 hours being suited.

After completion of the reaction, the manner of post-treatment differs depending upon whether the reaction product contains one or more carboxyl groups or one or more carboxylic ester groups. When the reaction product contains one or more carboxyl groups, an aqueous solution of a caustic alkali is poured into the reaction mixture to dilute the same such that the reaction product is dissolved in the aqueous alkaline solution, and the aqueous solution is then washed with a suitable solvent such as ethyl acetate or toluene.
Subsequently, hydrochloric acid is added to the separated water phase to acidify this water phase such that the compound containing the one or more carboxyl groups (hereinafter called "the carboxyl-containing compound" for the sake of brevity) is liberated. The thus-liberated, carboxyl-containing compound is collected by filtration, thoroughly washed with distilled water, and then dried to afford the target carboxyl-containing compound.

When the reaction product contains one or more carboxylic ester groups, a mineral acid such as hydrochloric acid is added to the reaction mixture to neutralize the same, and the reaction product is then extracted in a suitable solvent such as ethyl acetate. The solvent phase is washed with distilled water, and the solvent is then distilled off to obtain a crude product containing the one or more carboxylic ester groups. The crude product is recrystallized from a solvent, suitably from an alcoholic solvent such as methanol or isopropyl alcohol to afford the target compound containing the one or more carboxylic ester groups.

The present invention will next be described in more detail based on Examples. It should however be borne in mind that the present invention is by no means limited by or to the following Examples.

### Example 1

Charged into a 500-mL flask equipped with a stirrer, a thermometer and a dropping funnel were indene (purity: 96 wt.%, 50 g, 0.413 mole), dioxane (200 g), 40 wt.% benzyltrimethylammonium hydroxide (5 g, 12 mmol) and sodium methoxide (0.16 g, 3 mmol). The internal temperature was controlled to 30°C, followed by the addition of acrylic acid (7.4 g, 0.103 mole) over about 2 hours while externally cooling the flask to maintain the internal temperature at the same temperature.

After the indene and acrylic acid were allowed to react further at the same temperature for 10 hours, a 5 wt.% aqueous solution of sodium hydroxide (1 L) was poured into the reaction mixture to dilute the same. The resulting aqueous sodium hydroxide solution was washed twice with ethyl acetate (200 mL). Subsequently, concentrated hydrochloric acid was added to the water phase to acidify the same such that the carboxyl-containing compound was liberated. The thus-liberated product was collected by filtration, thoroughly washed with distilled water, and then dried to afford 3',3'-(3-indenylidene)-dipropionic acid represented by the following formula (11.0 g, yield: 82.0% based on the acrylic acid).

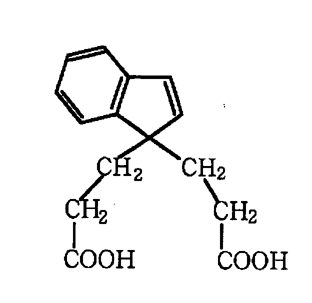

### Example 2

Charged into a 500-mL flask equipped with a stirrer, a thermometer and a dropping funnel were indene (purity: 96 wt.%, 50 g, 0.413 mole), dioxane (200 g), 40 wt.% benzyltrimethylammonium hydroxide (5 g, 12 mmol) and sodium methoxide (0.16 g, 3 mmol). The internal temperature was controlled to 30°C, followed by the addition of methyl acrylate (8.9 g, 0.103 mole) over about 2 hours while externally cooling the flask to maintain the internal temperature at the same temperature.

After the indene and methyl acrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford dimethyl 3',3'-(3-indenylidene)dipropionate represented by the following formula (13.0 g, yield: 87.1% based on the methyl acrylate).

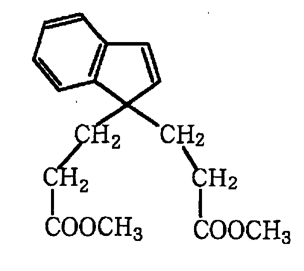

### Example 3

Charged into a 500-mL flask equipped with a stirrer, a thermometer and a dropping funnel were indene (purity: 96 wt.%, 50 g, 0.413 mole), dioxane (200 g), 40 wt.% benzyltrimethylammonium hydroxide (5 g, 12 mmol) and sodium methoxide (0.16 g, 3 mmol). The internal temperature was controlled to 30°C, followed by the addition of methyl methacrylate (10.3 g, 0.103 mole) over about 2 hours while externally cooling the flask to maintain the internal temperature at the same temperature.

After the indene and methyl methacrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford 3',3'-(3-indenylidene)di(methyl 2'-methylpropionate) represented by the following formula (14.1 g, yield: 86.6% based on the methyl methacrylate).

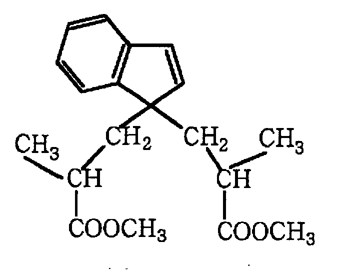

### Example 4

Charged into a 500-mL flask equipped with a stirrer, a thermometer and a dropping funnel were indene (purity: 96 wt.%, 50 g, 0.413 mole), dioxane (200 g), 40 wt.% benzyltrimethylammonium hydroxide (5 g, 12 mmol) and sodium methoxide (0.16 g, 3 mmol). The internal temperature was controlled to 30°C, followed by the addition of glycidyl methacrylate (14.6 g, 0.103 mole) over about 2 hours while externally cooling the flask to maintain the internal temperature at the same temperature.

After the indene and glycidyl methacrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford 3',3'-(3-indenylidene)di((2",3"-epoxypropyl) 2'-methylpropionate) represented by the following formula (17.3 g, yield: 83.8% based on the glycidyl methacrylate).

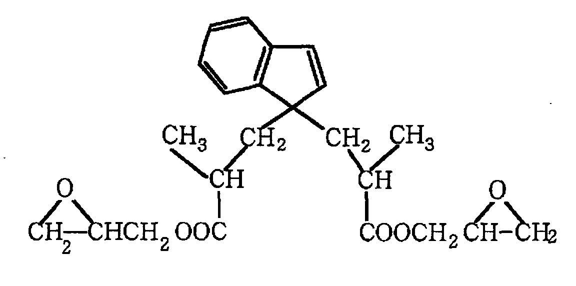

### Example 5

Charged into a 500-mL flask equipped with a stirrer, a thermometer and a dropping funnel were fluorene (purity: 95 wt.%, 50 g, 0.286 mole), dioxane (200 g), 40 wt.% benzyltrimethylammonium hydroxide (5 g, 12 mmol) and sodium methoxide (0.33 g, 6.1 mmol). The internal temperature was controlled to 30°C, followed by the addition of acrylic acid (47.7 g, 0.662 mole) over about 2 hours while externally cooling the flask to maintain the internal temperature at the same temperature.

After the fluorene and acrylic acid were allowed to react further at the same temperature for 15 hours, a 5 wt.% aqueous solution of sodium hydroxide (2 L) was poured into the reaction mixture to dilute the same. The resulting aqueous sodium hydroxide solution was washed twice with ethyl acetate (300 mL). Subsequently, concentrated hydrochloric acid was added to the water phase to acidify the same such that the carboxylic acid compound was liberated. The thus-liberated carboxylic acid compound was collected by filtration, thoroughly washed with distilled water, and then dried to afford 3',3'-(9-fluorenylidene)dipropionic acid represented by the following formula (80.3 g, yield: 90.4% based on the fluorene).

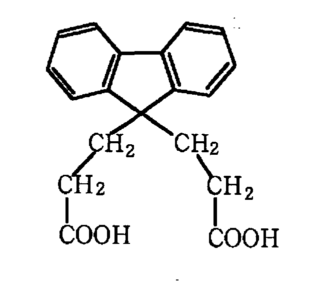

### Example 6

Charged into a 500-mL flask equipped with a stirrer, a thermometer and a dropping funnel were fluorene (purity: 95 wt.%, 50 g, 0.286 mole), dioxane (200 g), 40 wt.% benzyltrimethylammonium hydroxide (5 g, 12 mmol) and sodium methoxide (0.33 g, 6.1 mmol). The internal temperature was controlled to 30°C, followed by the addition of methyl acrylate (56.9 g, 0.662 mole) over about 2 hours while externally cooling the flask to maintain the internal temperature at the same temperature.

After the indene and methyl acrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford dimethyl 3',3'-(9-fluorenylidene)dipropionate represented by the following formula (89.1 g, yield: 92.2% based on the fluorene).

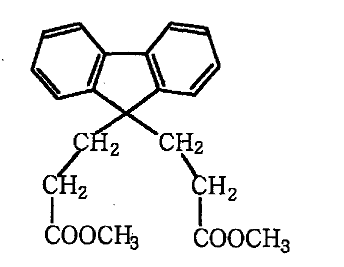

### Example 7

Charged into a 500-mL flask equipped with a stirrer, a thermometer and a dropping funnel were fluorene (purity: 95 wt.%, 50 g, 0.286 mole), dioxane (200 g), 40 wt.% benzyltrimethylammonium hydroxide (5 g, 12 mmol) and sodium methoxide (0.33 g, 6.1 mmol). The internal temperature was controlled to 30°C, followed by the addition of methyl methacrylate (66.2 g, 0.662 mole) over about 2 hours while externally cooling the flask to maintain the internal temperature at the same temperature.

After the indene and methyl methacrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford 3',3'-(9-fluorenylidene)di(methyl 2'-methylpropionate) represented by the following formula (95.2 g, yield: 90.9% based on the fluorene).

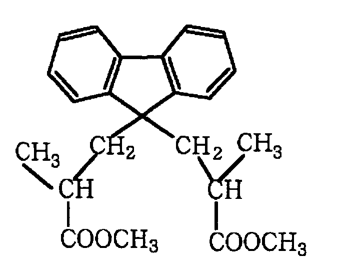

### Example 8

Charged into a 500-mL flask equipped with a stirrer, a thermometer and a dropping funnel were fluorene (purity: 95 wt.%, 50 g, 0.286 mole), dioxane (200 g), 40 wt.% benzyltrimethylammonium hydroxide (5 g, 12 mmol) and sodium methoxide (0.33 g, 6.1 mmol). The internal temperature was controlled to 30°C, followed by the addition of glycidyl methacrylate (94.1 g, 0.662 mole) over about 2 hours while externally cooling the flask to maintain the internal temperature at the same temperature.

After the indene and glycidyl methacrylate were allowed to react further at the same temperature for 24 hours, 10 wt.% hydrochloric acid was added to the reaction mixture to neutralize the same, and water (1 L) was added further to dilute the reaction mixture. Subsequently, the reaction product was extracted with ethyl acetate (300 mL), the ethyl acetate phase was washed with water, and ethyl acetate was distilled off under reduced pressure. The resulting crude product was recrystallized from methanol to afford 3',3'-(9-fluorenylidene)di((2",3"-epoxypropyl) 2'-methylpropionate) represented by the following formula (108.5 g, yield: 84.3% based on the fluorene).

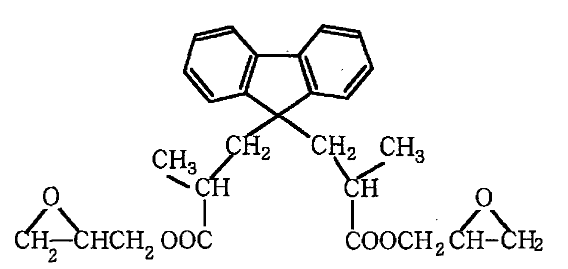

### Example 9

Charged into a 500-mL flask equipped with a stirrer, a thermometer and a dropping funnel were fluorene (purity: 80 wt.%, 50 g, 0.241 mole), dioxane (200 g), 40 wt.% benzyltrimethylammonium hydroxide (4.2 g, 10 mmol) and sodium methoxide (0.28 g, 5.1 mmol). The internal temperature was controlled to 30°C, followed by the addition of acrylic acid (40.2 g, 0.558 mole) over about 2 hours while externally cooling the flask to maintain the internal temperature at the same temperature.

After the fluorene and acrylic acid were allowed to react further at the same temperature for 15 hours, a 5 wt.% aqueous solution of sodium hydroxide (2 L) was poured into the reaction mixture to dilute the same. The resulting aqueous sodium hydroxide solution was washed twice with ethyl acetate (300 mL). Subsequently, concentrated hydrochloric acid was added to the water phase to acidify the same such that the carboxylic acid compound was liberated. The thus-liberated carboxylic acid compound was collected by filtration, thoroughly washed with distilled water, and then dried to afford 3',3'-(9-fluorenylidene)dipropionic acid represented by the following formula (58.2 g, purity: 98.2%, yield: 76.5% based on the fluorene).

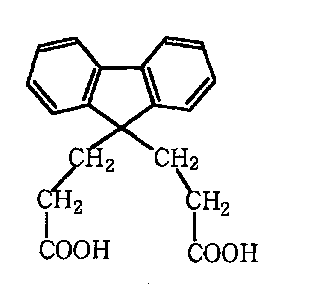

In a similar manner as in Examples 1-9, the following compounds were also synthesized.

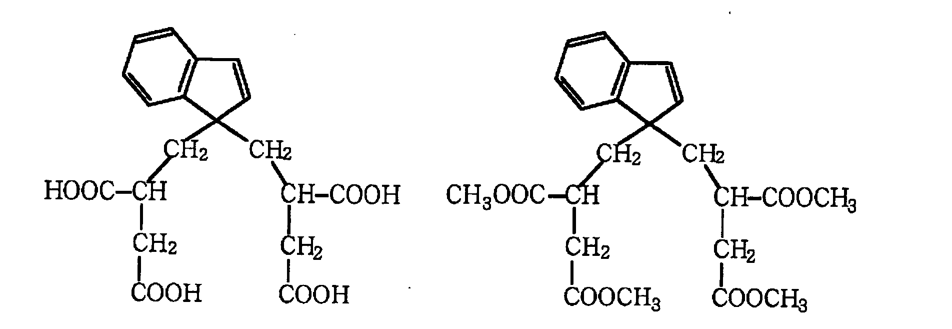

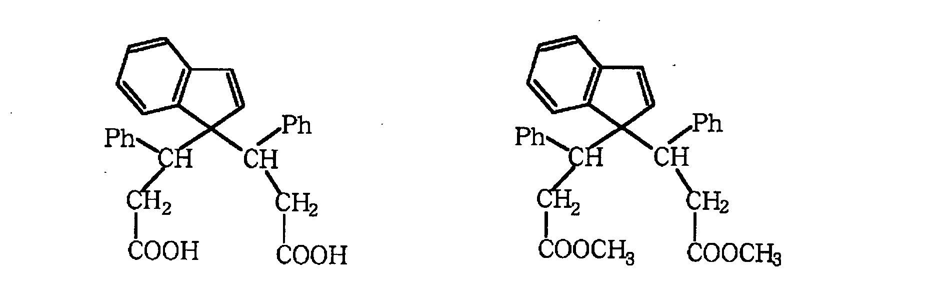

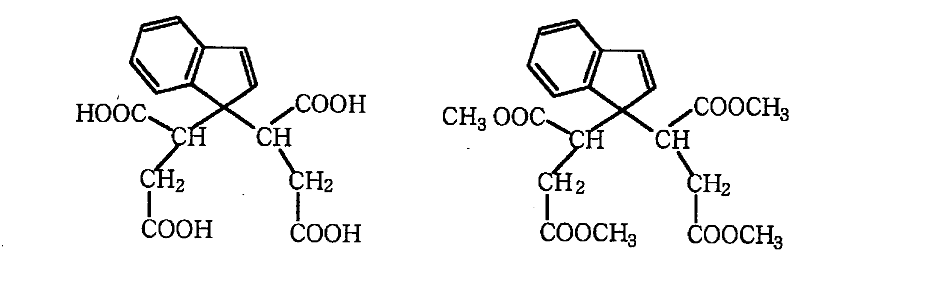

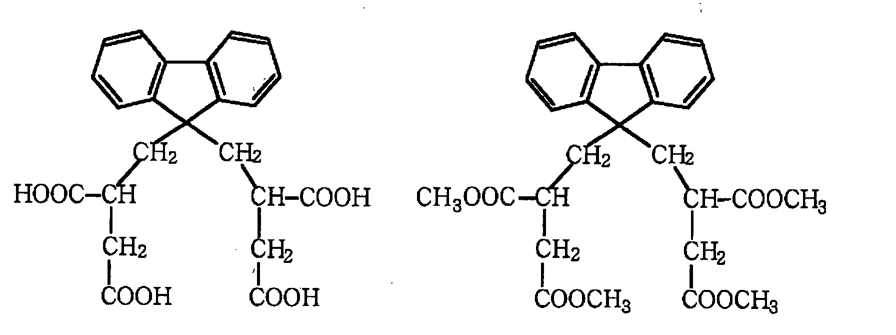

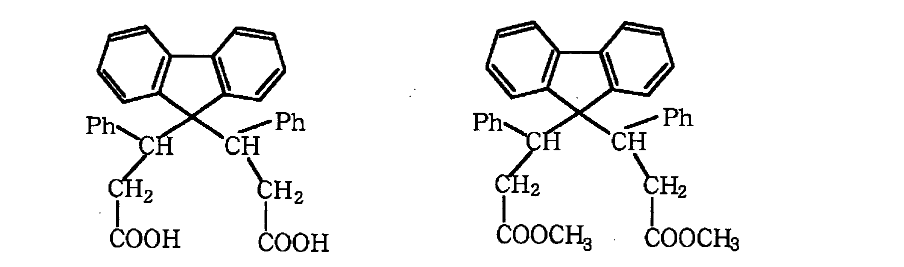

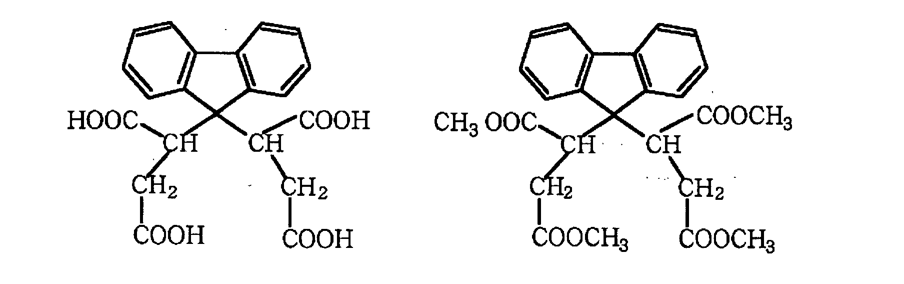

## Claims

1. A process for the preparation of a carboxylic acid compound having an indene skeleton and represented by the following formula (2), **characterized by** reacting an unsaturated carboxylic acid compound represented by the following formula (1) with indene in the presence of a strong base catalyst: 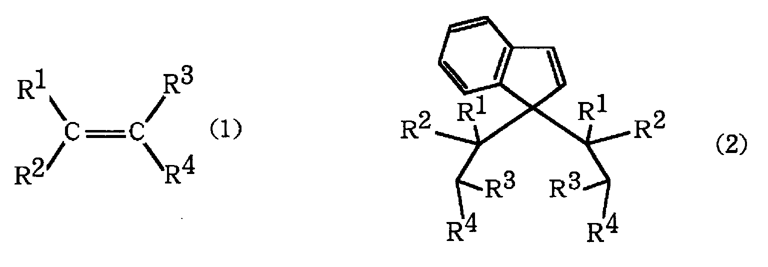 wherein R¹, R², R³ and R⁴ each independently represents an atom or group selected from a hydrogen atom, an alkyl group, an aryl group or a carboxyl group, with the proviso that at least one of R³ and R⁴ contains a carboxyl group or a salt thereof or a carboxylic ester group.

2. A process according to claim 1, wherein at least one of said alkyl group and aryl group contains an oxygen-containing group selected from a carboxyl group, a hydroxyl group or an ether group.

3. A process according to claim 1 or 2, wherein said unsaturated carboxylic acid compound represented by the formula (1) is (meth)acrylic acid, itaconic acid, cinnamic acid or maleic acid, fumaric acid or a salt or a C₁-C₄ mono- or dialkyl ester thereof.

4. A process according to claim 1, 2 or 3, wherein said unsaturated carboxylic acid compound is used in a proportion of from 0.05 to 4 moles per mole of said indene.

5. A process according to any one of claims 1-4, wherein said strong base catalyst is a quaternary ammonium hydroxide or an alkali metal alkoxide.

6. A process according to any one of claims 1-5, wherein said strong base catalyst is used in a proportion of from 0.5 to 10 mole % based on said indene.

7. A process for the preparation of a carboxylic acid compound having a fluorene skeleton and represented by the following formula (3), **characterized by** reacting an unsaturated carboxylic acid compound represented by the following formula (1) with fluorene in the presence of a strong base catalyst: 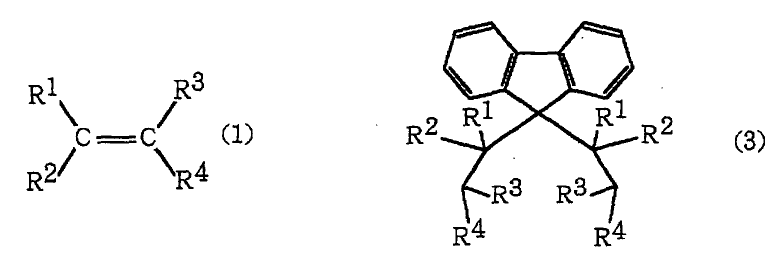 wherein R¹, R², R³ and R⁴ each independently represents an atom or group selected from a hydrogen atom, an alkyl group, an aryl group or a carboxyl group, with the proviso that at least one of R³ and R⁴ contains a carboxyl group or a salt thereof or a carboxylic ester group.

8. A process according to claim 7, wherein at least one of said alkyl group and aryl group contains an oxygen-containing group selected from a carboxyl group, a hydroxyl group or an ether group.

9. A process according to claim 7 or 8, wherein said unsaturated carboxylic acid compound represented by the formula (1) is (meth)acrylic acid, itaconic acid, cinnamic acid or maleic acid, fumaric acid or a salt or a C₁-C₄ mono- or dialkyl ester thereof.

10. A process according to claim 7, 8 or 9, wherein said unsaturated carboxylic acid compound is used in a proportion of from 2 to 10 moles per mole of said fluorene.

11. Aprocess according to any one of claims 7-10, wherein said strong base catalyst is a quaternary ammonium hydroxide or an alkali metal alkoxide.

12. A process according to any one of claims 7-11, wherein said strong base catalyst is used in a proportion of from 0.5 to 10 mole % based on said fluorene.
